# EUROPEAN PATENT APPLICATION

(11) **EP 0 943 332 A1**
(43) Date of publication of application: **22.09.1999**
(21) Application number: 98200893.0
(22) Date of filing: 19.03.1998
(51) Int. Cl.: A61K 31/35, A61K 31/575, A61K 31/20

(54) **Prevention and treatment of adhesions**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Kooistra, Teake, 2301 CE Leiden (NL); Goedde, Martin Franz, 20251 Hamburg (DE)
(74) Representative: Smulders, Theodorus A.H.J., Ir.

(57) **Abstract**

The invention relates to medical treatments and compositions to prevent or reduce the occurrence of adhesions to and between body organs, parts of body organs and/or tissues in animals, in particular human beings, such as the adhesions occurring after surgical procedures. The invention provides for reducing or preventing adhesions to or between organs, parts of organs or tissues, at a particular location, in a animal, comprising subjecting said animal to a treatment which provides for increased synthesis and/or secretion of plasminogen activator by mesothelial cells present at said location, or to a treatment which provides for decreased synthesis and/or secretion of plasminogen activator inhibitor by mesothelial cells present at said location.

## Description

### Field of the invention

The present invention relates to medical treatments to prevent or reduce the occurrence of adhesions to and between body organs, parts of body organs and/or tissues in animals, in particular human beings, such as the adhesions occurring after surgical procedures.

### Background of the invention

The occurrence of intraperitoneal adhesions after abdominal surgery or peritoneal inflammation is a common cause of intestinal obstruction and impaired fertility in women, and can be a complicating factor in subsequent operations. Although not all etiological aspects are known, the sequence of events leading to intraperitoneal adhesions and peritoneal fibrosis is thought to start with fibrin deposition followed by immigration and proliferation of fibroblasts and collagen synthesis by fibroblasts. The fibrin deposits are the result of an imbalance between the pro-coagulant (fibrin-forming) and the fibrinolytic (fibrin-dissolving) activities of the peritoneum. Adhesions may also occur after surgery of other body cavities, such as thorax or heart, and may occur after inflammatory processes in such cavities, such as seen with, for example, peritonitis, pericarditis and pleuritis.

It has been reasoned that the restoration of the balance at an early stage of the disease process might lead to diminished fibrin deposition and thus might retard or prevent the fibroblast response and, consequently, adhesion formation.

To prevent adhesions of peritoneal surfaces, various pharmacological agents have been investigated or proposed, including quinacrine hydrochloride (U.S. Patent No. 5,478,837), corticosteroids, non-steroidal anti-inflammatory drugs (Holz, Fertil. Steril. 37, 582, 1982), anticoagulants such as heparin (Jansen, Surg. Gynecol. Obstet. 166, 154, 1988), ibuprofen (U.S. Patent No. 4,346,108), antihistamines, antibiotics, dextran solutions, polyvinylpyrrolidone solutions and fibrinolytic agents (U.S. Patent No. 5,364,622).

With the exception of the fibrinolytic agents such as tissue-type plasminogen activator (t-PA), urokinase-type plasminogen activator (u-PA) and streptokinase, which appeared to be effective in animal models, results have so far been disappointing. These fibrinolytic agents convert the inactive zymogen plasminogen into plasmin. Plasmin effects the degradation of fibrin. Since fibrin deposition is a critical factor in the development of adhesions, administration of t-PA, u-PA or streptokinase results in the prevention of adhesion formation or removal of adhesions already formed.

This concept proved to be effective in the prevention of experimentally-induced peritoneal adhesions in animals, incl. rats (Evans et al., Am. J. Surg. 165, 229, 1993; Hill-West et al., J. Surg. Res. 59, 759, 1995), rabbits (Doody et al., Fertil. Steril. 51, 509, 1989; Menzies and Ellis, J. Royal. Soc. Med. 82, 534, 1989; Menzies and Ellis, Surg. Gynecol. Obstet. 172, 362, 1991; Dörr et al., Eur. J. Obstet. Gynecol. Reprod. Biol. 37, 287, 1990; Orita et al., Int. J. Fertil. 36, 172, 1991; dunn and Mohler, J. Surg. Res. 54, 242, 1993) and dogs (Am. J. Obstet. Gynecol. 165, 1539, 1991).

Limitations and drawbacks of the administration of fibrinolysis activators are the relatively large amounts required, a transient time of action due to leakage from the cavity to plasma (Flessner et al., Am. J. Physiol. 240, H15, 1985) or inhibition by specific inhibitors, and, in the case of streptokinase, the evoked immune response.

An attractive alternative would be to increase the endogenous fibrinolytic capacity of a cavity in a regulated way during a desired period of time, but no effective strategies have been described up till now.

Mesothelial cells are the major cell population involved in the lining of the peritoneal cavity. It has been shown that cultured human mesothelial cells (HMC) produce t-PA and u-PA, and that the activity of these plasminogen activators in the medium is counteracted by the concomitant secretion of specific plasminogen activator inhibitors, including plasminogen activator inhibitor-1 (PAI-1).

### Summary of the invention

The invention provides a method for reducing or preventing adhesions to or between organs, parts of organs or tissues, at a particular location, in an animal, comprising subjecting said animal to a treatment which provides for increased synthesis and/or secretion of plasminogen activator or for decreased synthesis and/or secretion of plasminogen activator inhibitor (PAI) by mesothelial cells present at said location.

Preferably, said animal is a mammal, most preferably a human being, and said location is or has been, or may become, exposed to trauma, which might lead to local fibrosis. In animals, the invention finds its use in veterinary practice, for example in small or large animal practice where birds, or mammalians or other vertebrate animals are treated. In humans, the invention finds its use in medicine. An example of trauma is a surgical procedure, e.g. abdominal or thoracic surgery. Another example is trauma after accidents, where for example internal bleeding is stopped by treatment with coagulants. After such a treatment with coagulants, the invention finds its use in helping prevent adhesions.

In a preferred embodiment, the invention provides a method wherein said treatment comprises administration to said animal of an active agent capable of increasing t-PA and/or u-PA synthesis and/or secretion or for decreasing synthesis and/or secretion of PAI-1 by said mesothelial cells. Said treatment comprises application of said active agent, preferably at said location after a risk for the occurrence of adhesions has been observed, but systemic or parenteral treatment is also possible, and such treatments may also be given as a preventive measure, for example shortly before a surgical treatment is being contemplated or going to be executed. As an example, the invention provides a method wherein said active agent is capable of enhancing t-PA synthesis or decreasing PAI-1 synthesis by mesothelial cells. Examples of such an active agent can be found in the group composed of vastatins such as simvastatin, lovastatin, mevinoline, distinct oxysterols such as 22(R)-hydroxycholesterol, retinoids such as all-trans retinoic acid and 9-cis retinoic acid.

In another preferred embodiment, the invention provides a method wherein said active agent is capable of inhibiting HMG-CoA reductase, thereby increasing t-PA and/or u-PA synthesis and/or secretion by mesothelial cells, and/or thereby decreasing synthesis and/or secretion of PAI-1 by these cells. Mevalonic acid (MVA) constitutes the key metabolite in the biosynthesis of isoprenoids, which are the precursors of sterols (e.g. cholesterol and oxysterols) and a variety of non-sterol derivatives such as farnesyl- and geranylgeranyl-pyrophosphate (GGPP). The production of MVA is catalysed by HMG-CoA reductase, inhibitors of which (vastatins such as simvastatin, lovastatin, mevinoline) are widely used as anti-atherosclerotic drugs in patients with hypercholesteremia. Other agents capable of acting synergetically with HMG-CoA redinctase inhibitors (thereby increasing t-PA synthesis and/or secretion or decreasing synthesis and/or secretion of PAI) are for example distinct natural oxysterols and 9-cis retinoic acid.

Inhibition of HMG-CoA reductase strongly increases t-PA synthesis in mesothelial cells, while decreasing PAI-1. For example, simvastatin treatment of such cells strongly increases t-PA synthesis, which, in a second aspect, is further potentiated by subsequent or co-administration of 9-cis retinoic acid and/or 22(R)-hydroxycholesterol, or combinations thereof.

The invention also provides a method which counteracts any of above said methods of treatment which provide for increased synthesis and/or secretion of plasminogen activator or decreased synthesis and/or secretion of plasminogen activator inhibitor in mesothelial cells. The invention thus not only provides a method to increase the endogenous fibrinolytic capacity of for example a cavity in a regulated way but also provides a method to increase said capacity for a derived period of time. In cases where one wishes to actively stop or mitigate any of above said treatments, it may be not enough to simply stop said treatment and wait till the effects dwindle down. A second treatment, with an active agent capable of counteracting the first treatment is provided by the invention. The invention for example provides an effective method to counteract the effect seen after treatment with HMG-CoA reductase inhibitors such as simvastatin, lovastatin, or mevinoline or for example with distinct oxysterols and retinoic acid, by, for example subsequent or concomitant, administration of for example MVA or geranylgeraniol, which quench or counteract a plasminogen activator increase or PAI decrease.

The invention also provides use of an active agent capable of providing for increased synthesis and/or secretion of plasminogen activator or decreased synthesis and/or secretion of plasminogen activator inhibitor in mesothelial cells, for preparing a pharmaceutical composition for reducing or preventing adhesions to or between organs, parts of organs or tissues. A preferred embodiment of the invention is wherein said mesothelial cells are mammalian, for example human.

In one embodiment of the invention it is preferred that said active agent is capable of enhancing synthesis and/or secretion of plasminogen activator, preferably of t-PA, by said mesothelial cells. In yet another embodiment of the invention it is preferred that said active agent is capable of decreasing synthesis and/or secretion of plasminogen activator inhibitor, preferably of PAI-1, by said mesothelial cells. According to preferred embodiments, said active agent is selected from the group composed of vastatins such as simvastatin, lovastatin, mevinoline, distinct oxysterols such as 22(R)-hydroxycholesterol, and 9-cis retinoic acid. Preferably, said active agent is selected from the group consisting of inhibitors of mevalonic acid synthesis such as simvastatin or a functional equivalent thereof, inhibitors of geranylgeraniol synthesis or oxysterols such as 22(R)-hydroxycholesterol or functional equivalents thereof.

In another embodiment, said active agent is selected from agents or compositions capable of inhibiting HMG-CoA reductase or from agents or compositions capable of activating liver-X-receptor or related receptors. Liver-X-receptor (LXR) is an orphan receptor that displays constitutive transcriptional activity. For example, naturally occurring oxysterols such as 20(S)-hydroxycholesterol ((OH-Chol), 22(R)-OH-Chol, 24(S)-OH-Chol and 24(S), 25-epoxy-Chol, but not other products of mevalonate metabolism, activate LXR at concentrations consistent with those found in tissue extracts. In contrast, GG-PP, another metabolite of MVA, is a strong inhibitor of LXR. A software-based search of the t-PA promoter for a binding site for LXR led to the identification of a putative response element at position -8,682: ACTGGG taac ACTTGG (motif DR4/inv).

The invention also provides use of an active agent capable of counteracting increased synthesis and/or secretion of plasminogen activator or decreased synthesis and/or secretion of plasminogen activator inhibitor in mesothelial cells, for preparing a pharmaceutical composition for counteracting or mitigating the action of any of the above described pharmaceutical compositions for reducing or preventing adhesions to or between organs, parts of organs or tissues, said active agent preferably being MVA or geranylgeraniol.

The compositions and methods according to the present invention are useful in reducing, minimising or preventing adhesion formation, the most common cause of which is prior surgery. The compositions and methods according to the present invention have been shown to be especially effective in preventing the formation of adhesions between organ surfaces, in particular adhesion formation in the peritoneum following surgery. The present invention also finds utility in other contexts, however, such as e.g. for cardiovascular, orthopaedic, thoracic, ophthalmic, central nervous system and other uses where prevention of the formation of adhesions after surgery, or during or after inflammatory processes is a significant concern. In the following discussion, attention is directed primarily to a description of compositions and methods useful in inhibiting peritoneal adhesion formation, but the various possibilities and preferred embodiments disclosed therein apply to all other conditions where the invention is applicable.

The term "pharmaceutical composition" as used herein is intended to cover both the active agent alone and a composition containing the active agent together with a pharmaceutically acceptable carrier, diluent or excipient.

Pursuant to one embodiment of the present invention, an active agent is administered in an effective concentration at the site of potential adhesion formation. The administration may be done as a single dose, as a discontinuous sequence of various doses, or continuously for a period of time sufficient to permit substantial tissue repair, in particular re-epithelisation or mesothelial repair. The active agent is typically administered during the surgical procedure up to some time after completion thereof, and administration may even be carried out or initiated shortly before surgery. In the case of a continuous administration, the duration of the administration may vary depending upon a number of factors which would readily be appreciated by those skilled in the art. In general, the administration of a composition in accordance with the present invention should be effected from the time of surgery for at least one or two days after completion of the surgical procedure. As healing is in most cases complete within about two weeks, it is generally not necessary to continue administration of a composition in accordance with the present invention much longer than two weeks. Preferably, a composition according to the present invention is administered from about the time of surgery for a period of about one day to about two weeks, such as more particularly for a period of about two days to about one week.

The administration dose of the active agent may be varied over a fairly broad range. The concentrations of an active agent which can be administered would be limited by efficacy at the lower end and the solubility of the compound at the upper end. The optimal dose or doses for a particular patient should and can be determined by the physician or medical specialist involved, taking into consideration well known relevant factors such as the condition, weight and age of the patient, the given or predicted extent of the adhesions, etc.

The active agent may be administered directly in a suitable vehicle, such as e.g. phosphate-buffered saline (PBS) or as solutions in alcohol or DMSO. Pursuant to preferred embodiments of the present invention, however, the active agent is administered through a single dose delivery using a drug-delivery system, such as a sustained-release delivery system, which enables the maintenance of the required concentrations of the active agent for a period of time sufficient for adequate tissue repair. A suitable drug-delivery system would be pharmacologically inactive or at least tolerable. It should preferably not be immunogenic nor cause inflammatory reactions, and should permit release of the active agent so as to maintain effective levels thereof over the desired time period. A large variety of alternatives are known in the art as suitable for purposes of sustained release and are contemplated as within the scope of the present invention. Suitable delivery vehicles include, but are not limited to, the following: microcapsules or microspheres; liposomes and other lipid-based release systems; viscous instillates; absorbable and/or biodegradable mechanical barriers and implants; and polymeric delivery materials, such as polyethylene oxide/polypropylene oxide block copolymers, polyesters, cross-linked polyvinylalcohols, polyanhydrides, polymethacrylate and polymethacrylamide hydrogels, anionic carbohydrate polymers, etc. useful delivery systems are well known in the art.

A highly suitable formulation to achieve the active agent release comprises injectable microcapsules or microspheres made from a biodegradable polymer, such as poly(dl-lactide), poly(dl-lactide-co-glycolide), polycaprolactone, polyglycolide, polylactic acid-co-glycolide, poly(hydroxybutyric acid), polyesters or polyacetals. Injectable systems comprising microcapsules or microspheres having a diameter of about 50 to about 500 micrometers offer advantages over other delivery systems. For example, they generally use less active agent and may be administered by paramedical personnel. Moreover, such systems are inherently flexible in the design of the duration and rate of separate drug release by selection of microcapsule or microsphere size, drug loading and dosage administered. Further, they can be successfully sterilized by gamma irradiation.

The design, preparation and use of microcapsules and microspheres are well within the reach of persons skilled in the art and detailed information concerning these points is available in the literature. Biodegradable polymers (such as lactide, glycolide and caprolactone polymers) may also be used in formulations other than microcapsules and microspheres; e.g. pre-made films and spray-on films of these polymers containing the active agent would be suitable for use in accordance with the present invention. Fibres or filaments comprising the active agent are also contemplated as within the scope of the present invention.

Another highly suitable formulation for a single-dose delivery of the active agent in accordance with the present invention involves liposomes. The encapsulation of an active agent in liposomes or multilamellar vesicles is a well known technique for targeted drug delivery and prolonged drug residence. The preparation and use of drug-loaded liposomes is well within the reach of persons skilled in the art and well documented in the literature.

Yet another suitable approach for single dose delivery of an active agent in accordance with the present invention involves the use of viscous installates. In this technique, high molecular weight carriers are used in admixture with the active agent, giving rise to a structure which produces a solution with high viscosity. Suitable high molecular weight carriers include, but are not limited to, the following: dextrans and cyclodextrans; hydrogels; (cross-linked) viscous materials, including (cross-linked) viscoelastics; carboxymethylcellulose; hyaluronic acid; and chondroitin sulfate. The preparation and use of drug-loaded viscous instillates is well known to persons skilled in the art.

Pursuant to yet another approach, the active agent may be administered in combination with absorbable mechanical barriers such oxidised regenerated cellulose. The active agent may be covalent or non-covalently (e.g. ionically) bound to such a barrier, or it may simply be dispersed therein.

The above described approaches for administration apply in general to any active agent in accordance with the present invention. It, however, may be preferred to treat or prevent the occurrence of adhesions with compositions that are delivered via a sustained-release delivery system, whereas counteracting or mitigating such a treatment may preferably be achieved via administration of compositions that release the active agent rapidly, and thus allow immediate action.

### Brief description of the drawings

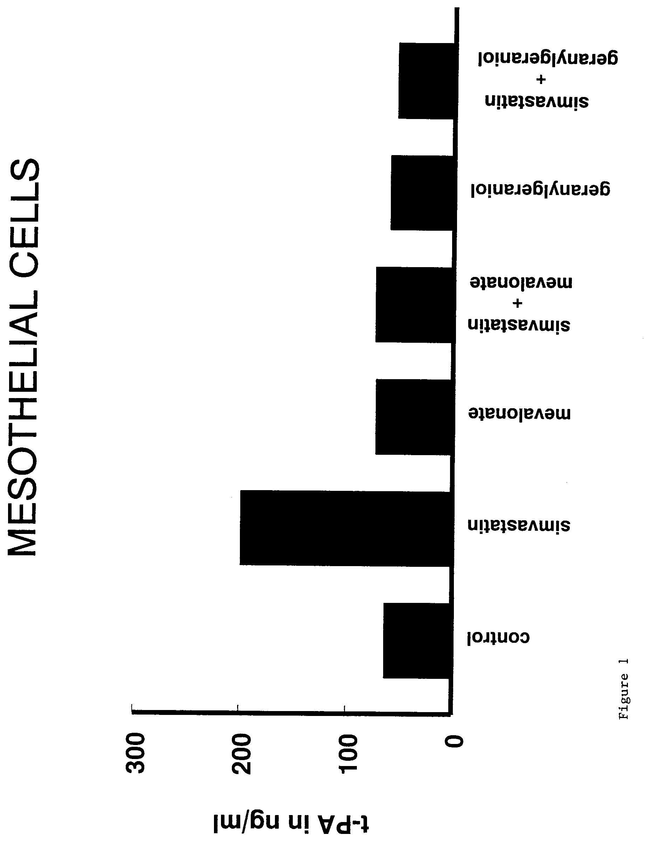

Fig. 1. Human mesothelial cells were incubated for 24 hours at 37°C with vehicle only (control) or 1 µmol/L simvastatin, 10 µmol/L mevalonate, 10 µmol/L geranylgeraniol or combinations thereof. The conditioned media were analysed for t-PA antigen. The data shown are from one representative experiment performed in triplicate and are expressed as mean values.

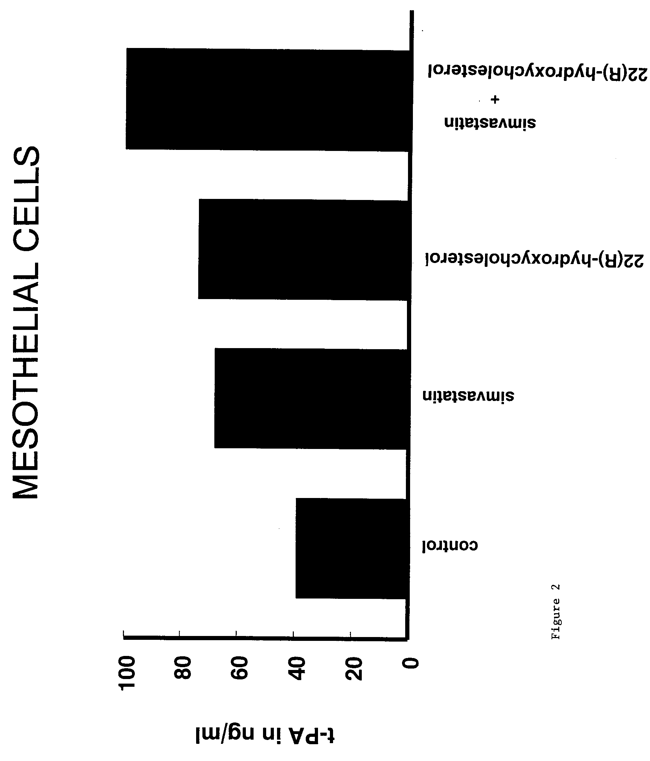

Fig. 2. Human mesothelial cells were incubated for 24 hours at 37°C with vehicle only (control) or 1 µmol/L simvastatin, 10 µmol/L 22(R)-hydroxycholesterol, or combinations thereof. The conditioned media were analysed for t-PA antigen. The data shown are from one representative experiment performed in triplicate and are expressed as mean values.

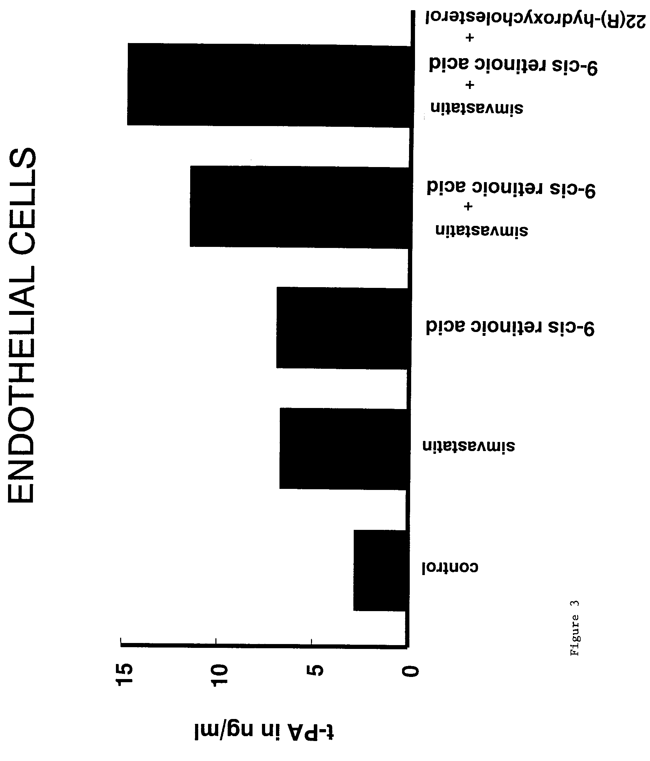

Fig. 3. Human endothelial cells were incubated for 24 hours at 37°C with vehicle only (control) or 1 µmol/L simvastatin, 10 µmol/L 9-cis retinoic acid. The conditioned media were analysed for PAI-1 antigen. The data shown are from one representative experiments performed in triplicate and are expressed as mean values.

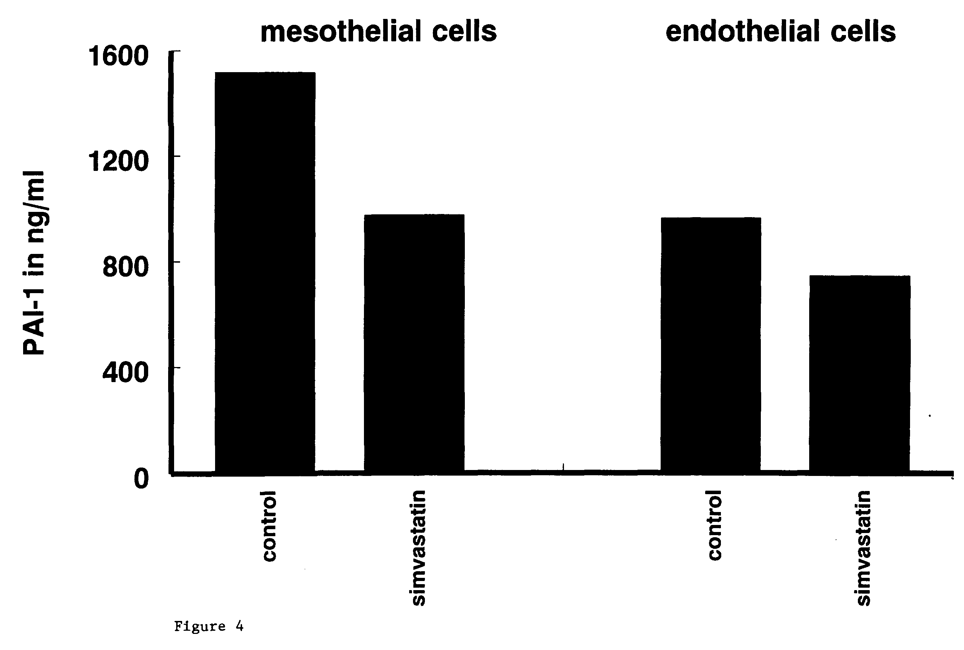

Fig. 4. Human mesothelial cells and human endothelial cells were incubated for 24 hours at 37°C with vehicle only (control) or 1 µmol/L simvastatin, 10 µmol/L 9-cis retinoic acid, 10 µmol/L 22(R)-hydroxycholesterol, or combinations thereof. The conditioned media were analysed for t-PA antigen. The data shown are from one representative experiment performed in triplicate and are expressed as mean values.

### Experimental part

We have discovered that t-PA synthesis in HMC is both positively and negatively regulated by distinct products of mevalonic acid metabolism. By inhibiting endogenous mevalonic acid synthesis, we were able to increase endogenous t-PA production by HMC. The addition of certain oxysterols further increased this stimulated t-PA expression, whereas the administration of geranylgeraniol, another metabolite of mevalonic acid, suppressed the elevated t-PA expression observed in HMC with diminished mevalonic acid biosynthesis. Comparable results were obtained with other t-PA producing cells, including human vascular endothelial cells.

The use of specific mevalonic acid metabolites such as certain oxysterols and/or inhibitors of mevalonic acid synthesis or the formation of certain mevalonic acid metabolites such as geranylgeraniol provides the possibility to increase the endogenous fibrinolytic capacity of human mesothelial cells and other t-PA-producing cells where such an increased capacity is desirable.

Multiple studies were performed to demonstrate that blocking of mevalonate biosynthesis and/or addition of 22(R)-hydroxycholesterol leads to increases in the levels of t-PA in the conditioned media of mesothelial cells (MC) or endothelial cells (EC). The model systems used consisted of cultured human peritoneal mesothelial cells (HMC) which were isolated from the omental tissue of consenting patients undergoing elective surgery and of cultured human endothelial cells (HEC) which were isolated from human umbilical cord veins. Cells were grown in fibrinonectin-coated dishes in M199 supplemented with 20 mmol/L HEPES (pH 7,4), 10% (vol/vol), human serum, 10% (vol/vol) heat-inactivated newborn calf serum, 150 µg/mL endothelial cell growth supplement, 2 mmol/L L-glutamine, 5 IU/mL heparin, 100 U/mL penicillin, and 100 µg/mL streptomycin at 37 °C in a 5% CO₂/95% air atmosphere. The medium was replaced every 2 to 3 days. Subcultures were obtained by trypsin/EDTA treatment of confluent monolayers at a split ratio of 1:3. Cells from omental tissue were pure mesothelial cells as assessed by their uniform cobblestone appearance at confluence, by the absence of von Willebrand factor, and the uniform positive staining for cytokeratins 8 and 18 and for vimentin (van Hinsbergh et al., Blood 75, 1490, 1990). Cells from umbilical cord veins were pure endothelial cells as assessed by their uniform cobblestone appearance at confluence and the uniform positive staining for von Willebrand factor (Jaffe et al., J. Clin. Invest. 52, 2745, 1973 Van Hinsbergh et al., Arteriosclerosis 3, 547, 1983). For the experiments, confluent cultures were used at the second or third passage, and cells were always re-fed the day before the experiment with incubation medium: M199, supplemented with 20 mmol/L HEPES (pH 7.4), 10% (vol/vol) human serum, 2 mmol/L-glutamine, and antibiotics. Conditioned media were obtained by incubating cells in 2 cm² dishes at 37°C with 0.5 mL incubation medium containing the appropriate concentration of the test compound or stock solvent. The conditioned media were centrifuged for 5 minutes at 8,000 rpm in a Microfuge centrifuge to remove cells and cellular debris. Conditioned media were stored at -20°C until use.

### Analysis of metabolites of the mevalonate (MVA) pathway

MVA is a common metabolite used by virtually all eukaryotic cells. MVA metabolism yields a series of isoprenoids that serve as precursors to a large number of important molecules, including cholesterol, oxysterols, farnesyl-pyrophosphate (F-PP) and geranylgeranyl-pyrophosphate (GG-PP). We have established procedures for thin-layer chromatography- and HPLC-based analysis and quantification of intermediates of the MVA pathway and oxysterols (i.e. hydroxylated derivates of cholesterol) from cells that have been administered radiolabelled precursors such as [¹⁴C] acetate or [¹⁴C] MVA. Interestingly, we have found that t-PA synthesis in HMC is positively and negatively regulated by distinct products of MVA metabolism. Our experiments indicate that these effects are likely to be mediated via a recently discovered orphan member of the nuclear hormone receptor superfamily, such as liver-X-receptor (LXR). Conditions that stimulate or quench the transactivation activity of LXR in an artificial model system, proved to evoke parallel changes in t-PA expression in our HMC. 1. Inhibition of endogenous MVA synthesis in HMC strongly increased t-PA gene expression. 2. Addition of specific oxysterols, shown to activate LXR, increased t-PA expression under basal conditions and further increased the stimulated t-PA expression seen in MVA-deprived HMC. 3. The effect of oxysterols (ligands for LXR) was potentiated by 9-cis retinoic acid, a ligand for retinoid-X-receptor (RXR) which is the heterodimer partner of LXR. 4. The administration of geranylgeraniol, another metabolite of MVA, suppressed the elevated t-PA expression in MVA-deprived HMC, whereas farnesol was without effect. 5. LXR-RXR heterodimers bind to specific nucleotide sequences, response elements, only present in target genes; t-PA contains such a specific response element (see below).

The activation profile of t-PA and LXR clearly differs from that of related receptors such as the farnesol-X-receptor (Cell 81, 687-693, 1995) and sterol receptors described by Goldstein and Brown (Cell 89, 331-340, 1997). Of interest is also our finding that inhibition of endogenous MVA synthesis in HMC suppressed significantly PAI-1 synthesis, indicating that MVA metabolites have a more general effect on endothelial functioning than merely changing t-PA synthesis.

### Oxysterols and fibrinolysis

It should be noted that the specific group of oxygenated cholesterol derivatives ('oxysterols") that activate transcription of target genes through the nuclear receptor LXR (see below) differ clearly from the atherogenic oxysterols present in oxidised low-density lipoprotein (Ox-LDL), and which are auto-oxidation products of cholesterol. Ox-LDL, an atherogenic lipoprotein that exists in atherosclerotic vessel walls, has been shown to stimulate PAI-1 production while it inhibits t-PA release from cultured humn endothelial cells (HEC). These effects of Ox-LDL could be mimicked by certain oxysterols, notably 25(R)-hydroxycholesterol and 7-ketocholesterol, which are formed during oxidation of LDL.

In contrast to the Ox-LDL-derived oxysterols, we have found that other, naturally occurring, intra-cellularly formed oxystereols such as 22(R)-hydroxycholesterol specifically stimulate t-PA synthesis in HMC. Also, we observed that precursors along the main pathway to cholesterol synthesis both positively and negatively regulate t-PA and PAI-1 synthesis in HMC. These results suggest a novel interplay between intermediates of cholesterol biosynthesis and metabolites thereof on the one hand and mesothelial functioning on the other hand.

### Nuclear hormone receptors and LXR

A variety of cholesterol derivatives, including steroid hormones and vitamin D, exert effects on gene expression through interactions with members of the nuclear receptor superfamily. Members of this family function as ligand-activated transcription factors by binding to short stretches of DNA, termed hormone response elements, present in the regulatory regions of target genes. In addition to the nuclear receptors with known ligands, this superfamily comprises a large number of structurally related members that contain DNA binding domains and putative ligand binding domains, but lack identified ligands, the so-called "orphan receptors". Liver-X-receptor (LXR) is an orphan receptor that displays constitutive transcriptional activity. Naturally occurring oxysterols such as 20(S)-hydroxycholesterol ((OH-Chol), 22(R)-OH-Chol, 24(S)-OH-Chol and 24(S), 25-epoxy-Chol, but not other products of mevalonate metabolism, activated LXR at concentrations consistent with those found in tissue extracts. In contrast, GG-PP, another metabolite of MVA, was a strong inhibitor of LXR. LXR binds as a heterodimer with the retinoid-X-receptor (RXR) to DENA response elements that are direct repeats of the core sequence AG(G/T) TCA spaced by four nucleotides, a so-called DR-4 motif. Recently, it was found that two closely related LXR subfamily members exist, referred to as LXRα and LXRβ, which show very similar activation characteristics.

### LXR response element in the t-PA promoter

A software-based search of the t-PA promoter for a binding site for RXR/LXR led to the identification of a putative response element at position -8,682: ACTGGG taac ACTTGG (motif DR4/inv). A similar DR4 motif was shown to bind LXRα and LXRβ in series of gel retardation assays.

The sequence is part of a 2.4 kb DNA fragment, located 7.1 kb upstream relative to the start site of transcription, which acts as a multi-hormone enhancer.

### Analysis of intermediates of the mevalonate pathway

Procedures for the isolation and analysis of 11 intermediates of the mevalonate pathway from acetyl-CoA through geranylgeranyl-pyrophosphate were adapted from the literature (Anal. Biochem. 215, 141-149, 1993). Routinely, rapid suspension of the cells in neutral 7 M urea with subsequent freeze-lysing in liquid nitrogen was found to effectively extract the cells. Mg²⁺-dependent phosphohydrolases were inhibited by addition of EDTA. Extracts are partially purified by adsorption and desorption in high yield from an anion-exchange membrane. The pathway intermediates are subsequently separated and quantified by reversed-phase ion-pair HPLC by examining the incorporation of [¹⁴C]pyruvate, [¹⁴C]mevalonate or [³H]-isopentenyl-pyrophosphate as precursors. Identification is primarily on the basis of established retention times. The addition of internal standards at the beginning of the extraction procedure allows the correction of any variability in recovery of the radio-labelled intermediates between experiments or between individual samples. The procedure is readily adaptable to cultured human endothelial cells.

### Analysis of oxysterols

For the analysis of oxysterols, essentially the procedures described by the group of TA Spencer are used (J. Biol. Chem. 260, 14571-14579, 1985; 262, 14056-14062, 1987). After incubation of cells with [³H]mevalonate, sterol fractions of the cells are isolated and prepared for HPLC utilising procedures designed to prevent aut-oxidation of sterols and acid-catalysed hydrolysis of epoxides. Again, the addition of internal standards allows calculation of recoveries. Identification of the sterols will primarily be on the basis of HPLC retention times and co-chromatography with authentic samples. this procedure too is easily applicable to cultured mesothelial cells.

### Cell incubations and incubation variables

Throughout many experiments charcoal-treated or thoroughly extracted serum are used to remove sterols, hormones and lipids. In experiments where it is essential to deprive cells of exogenous cholesterol (to prevent oxysterol formation from existing cholesterol), cells are pre-treated with lipoprotein-depleted serum. Where appropriate both media and cells are collected for analysis.

Cells are compared for their mevalonate metabolites and sterols to evaluate differences.

In parallel, t-PA, u-PA, PAI-1 are measured to obtain information regarding the intracellular metabolic processes ("the isoprenoid balance") and the expression of markers of mesothelial functioning. Antigen measurements are performed using routine methods such as ELISA.

### Example 1

One way to increase t-PA synthesis by mesothelial cells is to prevent the intracellular synthesis of mevalonic acid (MVA) from 3-hydroxy-3-methylglutaryl coenzyme A (HMG-CoA), for example, by the administration of simvastatin, an inhibitor of HMG-CoA reductase (Goldstein and Brown, Nature 343, 425, 1990).

The results shown in Figure 1 demonstrate that addition of simvastatin (1 µmol/L) increased t-PA levels in 24 hours-conditioned medium from 64 ng/ml under control conditions to 199 ng/ml in the presence of simvastatin. Co-administration of mevalonate (10 µmol/L) or geranylgeraniol (10 µmol/L), compounds which by themselves had little effect on t-PA synthesis, largely counteracted the t-PA synthesis stimulating effect of simvastatin.

### Example 2

We measured t-PA antigen accumulation in the conditioned media of mesothelial cells after 24 hours incubation in the presence of simvastatin (1 µmol/L), 22(R)-hydroxycholesterol (10 µmol) or a combination thereof (Fig. 2). Both compounds increased t-PA antigen production and, when added together, showed at least additive effects.

### Example 3

To check whether inhibition of intracellular MVA synthesis also increases t-PA synthesis in cells other than mesothelial cells, we measured t-PA antigen accumulation in the conditioned media of endothelial cells after 24 hours incubation in the presence of simvastatin (1 µmol/L). We found that simvastatin increased t-PA levels to 6.7 ng/ml as compared to 2.8 ng/ml under control conditions (Fig. 3). The effect of simvastatin was further potentiated by the simultaneous additionof 9-cis retinoic acid (10 µmol/L) and 22(R)-hydroxycholesterol (10 µmol/L).

### Example 4

To check whether the stimulating effect of simvastatin on t-PA synthesis in MEC and HEC is not the result of a general effect on protein synthesis in these cells, we also measured a specific inhibitor of t-PA, PAI-1. The addition of simvastatin (1 µmol/L) to MEC and HEC significantly reduced PAI-1 levels in 24 hours conditioned medium, as shown in Fig. 4.

## Claims

1. A method for reducing or preventing adhesions to or between organs, parts of organs or tissues, at a particular location, in an animal, comprising subjecting said animal to a treatment which provides for increased synthesis and/or secretion of plasminogen activator or for decreased synthesis and/or secretion of plasminogen activator inhibitor by mesothelial cells present at said location.

2. A method according to claim 1 wherein said animal is a mammal.

3. A method according to claim 2 wherein said mammal is a human being.

4. A method according to any of claims 1 to 3 wherein said location is, has been or may be exposed to trauma, which might lead to local fibrosis.

5. A method according to any of claims 1 to 4 wherein said trauma is a surgical procedure, e.g. abdominal or thoracic surgery.

6. A method according to any of claims 1 to 5 wherein said treatment comprises administration to said animal of an active agent capable of increasing plasminogen activator synthesis and/or secretion by said mesothelial cells.

7. A method according to claim 6 wherein said active agent is capable of increasing t-PA synthesis and/or secretion.

8. A method according to claim 6 or 7 wherein said active agent is selected from the group composed of vastatins such as simvastatin, lovastatin, mevinoline, distinct oxysterols such as 22(R)-hydroxycholesterol, and retinoic acid.

9. A method according to claim 6 or 7 wherein said active agent is capable of inhibiting HMG-CoA reductase or capable of activating liver-X-receptor.

10. A method according to any of claims 1 to 5 wherein said treatment comprises administration to said animal of an active agent capable of decreasing plasminogen activator inhibitor synthesis and/or secretion by said mesothelial cells.

11. A method according to claim 10 wherein said active agent is capable of decreasing PAI-1 synthesis and/or secretion.

12. A method according to claim 10 or 11 wherein said active agent is selected from the group composed of vastatins such as simvastatin, lovastatin, mevinoline.

13. A method which counteracts or mitigates a method according to any of claims 1 to 12 comprising subjecting said animal to a treatment with an active agent, said agent preferably being MVA or geranylgeraniol.

14. Use of an active agent capable of providing for increased synthesis and/or secretion of plasminogen activator or decreased synthesis and/or secretion of plasminogen activator inhibitor by mesothelial cells, for preparing a pharmaceutical composition for reducing or preventing adhesions to or between organs, parts of organs or tissues.

15. Use according to claim 14, wherein said mesothelial cells are mammalian, preferably human.

16. Use according to claim 14 or 15 wherein said active agent is capable of enhancing synthesis and/or secretion of plasminogen activator, preferably of t-PA.

17. Use according to claim 14 or 15 wherein said active agent is capable of decreasing synthesis and/or secretion of plasminogen activator inhibitor, preferably of PAI-1.

18. Use according to claim 16 or 17 wherein said active agent is selected from the group composed of vastatins, distinct natural oxysterols, and retinoic acid.

19. Use according to claim 16 or 17 wherein said active agent is capable of inhibiting HMG-CoA reductase or capable of activating liver-X-receptor.

20. Use of an active agent capable of controlling increased synthesis and/or secretion of plasminogen activator or decreased synthesis and/or secretion of plasminogen activator inhibitor in mesothelial cells, for preparing a pharmaceutical composition for counteracting or mitigating a method according to any of claims 1 to 12.

21. Use according to claim 20 wherein said active agent is MVA or geranylgeraniol.
